# EUROPEAN PATENT APPLICATION

(11) **EP 1 749 512 A2**
(43) Date of publication of application: **07.02.2007**
(21) Application number: 06118372.9
(22) Date of filing: 03.08.2006
(51) Int. Cl.: A61J 15/00

(54) **Method and apparatus for suctioning and refeeding gastric juices**

(30) Priority: 03.08.2005 US 197703
(71) Applicant: Moss, Gerald, White Plains NY 10605 (US)
(72) Inventor: Moss, Gerald, White Plains NY 10605 (US)
(74) Representative: Read, Matthew Charles

(57) **Abstract**

A decompressing and feeding method and system for safely feeding in the gastrointestinal tract (15) of a recovering patient (20) may continuously aspirate from the stomach and/or other portions of the digestive tract and feed at a rate commensurate with the ability of the intestines to absorb fluids including nutrient. Air is also aspirated in the process so that neither air nor excess fluids cause distension in the gastrointestinal tract. An aspirate (10) including digestive juices and nutrients from the stomach may be filtered and continuously refed together with unused feeding material into the gastrointestinal tract at a location that more efficiently moves and digests the food.

## Description

This invention generally relates to a device used to aspirate a gastro-intestinal tract and/or to deliver nutrients, fluids, medication, and/or aspirate into the gastro-intestinal tract. The invention specifically relates to devices and methods for safely, efficiently, and continuously aspirating from the stomach and feeding into the jejunum in the gastro-intestinal tract.

Frequently, hospital patients are unable to consume food normally. In these situations, it is often necessary to use a feeding tube to provide nutrition, fluids, and/or medicine. Such a tube is inserted into a patient's gastro-intestinal tract through the nose (nasogastric or nasoenteric tubes) or surgically by means of a gastrostomy or jejunostomy. Because adequate nutritional intake facilitates recovery, the proper use of a feeding tube and an associated device for feeding through the feeding tube can greatly benefit a patient.

However, the feeding tubes and the associated devices and methods also pose some discomfort, and even some potential risks, to patients. A number of commercially available feeding tubes exist. Unfortunately, all presently available feeding tube devices and methods for feeding through the tubes suffer from a variety of deficiencies. One common problem is that a feeding tube may deliver the fluids (which include liquid nutrition, hydrating fluids, and medicine to the patient) at a rate exceeding the ability of the gastro-intestinal tract to absorb such fluids. This results in an accumulation of fluid within the intestine. In particularly bad cases, accumulation of fluid causes distension of the intestine that leads to the temporary loss of all residual intestinal function. In rare cases, this intestinal distention may induce fatal vagal reflex circulatory changes. Approximately 1 in 1000 patients that are fed through a jejunal tube die of bowel necrosis, a complication that may be related to the lack of safety of the devices and methods of feeding directly into the gastro-intestinal tract. Severely ill, malnourished patients are most at risk for developing complications associated with overfeeding. Aside from being at high risk, these patients are also most in need of the earliest optimum nutrition. In any case, when a patient's intestine is not operating fully, then the amount of nutrition the patient can receive is limited. However, adequate nutrition is obviously a critical part of anyone's health and is necessary in order to provide optimum recovery for a patient. It is thus desirable to deliver as much nutrition to a patient as can be absorbed safely by the patient's impaired gastro-intestinal tract. Devices and methods of the past are deficient in providing this maximum feeding in a safe, prompt, and effective way to patients in need.

It is not desirable to simply deliver a maximum amount of nutrition into a patient's intestine. The reason for this is that overfeeding, which is delivering feedings at a rate exceeding the ability of the patient's gastro-intestinal tract to absorb its own secretions plus the added nutrition, itself presents serious hazards to a patient. Overfeeding a patient leads to an accumulation of fluid, which distends the intestine. This intestinal distension can lead to fatal circulatory changes. More commonly, complications from overfeeding include the temporary further impairment of intestinal function, with nausea and vomiting, which at a minimum causes discomfort and delays recovery. A common and more severe related complication is impaired ability to breathe deeply and cough because of abdominal distention, resulting in atelectasis and pneumonia.

A patient's impaired digestive system may also produce too many digestive secretions for the patient to reabsorb immediately. A typical person secretes seven to eight liters of fluid per day, starting with saliva. All secretions normally are reabsorbed by the intestine, without net loss or gain of fluid for the body. Unfortunately, during recovery from surgery the level of secretions remains relatively constant or decreases, while the intestine's ability to absorb secretions is typically impaired, sometimes severely. The result can be a build up of fluid in the intestine, with the same detrimental side effects as found with over-feeding. The problem of digestive secretions can be exacerbated by feeding.

When concentrated nutrition is delivered to a patient's gastro-intestinal tract, the body's natural response is to produce digestive secretions to dilute the feedings and break down the complex nutrients for absorption by the intestine. However, if a patient's gastro-intestinal function is severely impaired only a portion of the total fluid may be absorbed. By way of example, for 2 ml of nutrition delivered to the gastro-intestinal tract, 10 ml of digestive secretions may be provided in response. The intestine may temporarily be capable of absorbing only 2 ml of fluid, resulting in a net increase of 10 ml to the volume of fluid in the gastro-intestinal tract. Thus, nutrition delivered to the intestine may be competing with digestive secretions for absorption by the intestine. This problem may worsen as feedings continue, causing progressive intestinal distension which, in turn, further impairs intestinal function and reduces the amount of nutrient actually being absorbed. To avoid problems of overfeeding or excessive secretion build up during recovery, a patient's gastrointestinal tract may be aspirated to remove excessive fluid. However, any secretions removed in this way will cause a net loss of fluids and dehydration of the patient. Past solutions to this problem include providing replacement fluids in the form of an intravenous saline solution. However, this method of fluid replacement does not conserve nutrients and digestive juices that may have otherwise been used.

Another problem that has not adequately been solved is that the very presence of a nasal feeding tube stimulates swallowing by the patient, introducing additional air into the intestine. The presence of air within the gastro-intestinal tract interferes with the propulsion and absorption of nutrition and can be quite uncomfortable or painful to a patient. The use of an aspirating tube to remove air from the gastro-intestinal tract has been used to combat this problem. As set forth above, aspirating introduces additional problems of increased risks of dehydration and removal of potentially beneficial nutrients.

Unfortunately, the use of a second tube for aspiration may present additional problems. The introduction of a second tube may add to the pain and discomfort experienced by a patient. Furthermore, if a separate second tube is used, it may be difficult to effectively locate it proximate to the feeding tube, thereby preventing it from aspirating excessive food present in the gastro-intestinal tract.

This invention generally relates to methods and devices used to aspirate a gastro-intestinal tract and/or to deliver nutrients, fluids, medication, and/or aspirate into the gastro-intestinal tract. The invention specifically relates to methods and devices for safely, efficiently, and continuously aspirating from the stomach and feeding into the jejunum in the gastro-intestinal tract.

In a simple form, a method of feeding and decompressing in a gastrointestinal tract in accordance with the present invention may include the steps of feeding through a feeding channel into a jejunum and aspirating through an aspiration channel from a stomach. The step of feeding may include refeeding aspirate that has been removed from the stomach in the step of aspirating. The step of aspirating may include aspirating from the jejunum and the stomach.

The method of feeding and decompressing may include placing a distal end of a feeding channel at a first position in a jejunum, placing a distal end of an aspiration channel at a second position in the stomach, and aspirating via the aspiration channel. The method of feeding and decompressing, further may include the steps of placing a distal end of an aspiration channel at a position in the jejunum spaced proximally relative to the first position, and continuously aspirating and substantially collapsing a portion of a jejunum at the second position. It is to be understood that the step of feeding and the step of aspirating may include feeding and aspirating from separate tubes forming separate channels. Alternatively or additionally, the step of feeding and the step of aspirating may include feeding and aspirating through a single composite line having plural channels.

In another simple form, a method of feeding and decompressing in a gastrointestinal tract in accordance with the present invention may include the steps of placing a distal end of a feeding channel at a first position in the jejunum, placing a distal end of an aspiration channel at a second position in the stomach, and continuously aspirating via the aspiration channel. The steps of continuously aspirating and continuously feeding may further include alternatingly aspirating into a plurality of aspirate reservoirs in a repeated cycle. The method of feeding and decompressing in a gastrointestinal tract may further include continuously feeding via the feeding channel. The method of feeding and decompressing may include feeding from one of the plurality of aspirate reservoirs into which nothing is being aspirated. The step of continuously aspirating may further include aspirating an amount of aspirate greater than a predetermined maximum into a first overflow chamber connected to the first reservoir during a first part of a cycle, and aspirating an amount of aspirate greater than the predetermined maximum into a second overflow chamber connected to the second reservoir during a second part of the cycle.

The method of feeding and decompressing in a gastrointestinal tract may include inhibiting gastric reflux without permanently removing natural gastric juices. The method may include reducing one or more of dehydration, loss of natural antibodies, and lose of enzymes. The step of feeding may include refeeding a feeding material removed during the step of aspirating. The method may include filtering an aspirate from the stomach to a maximum particle size in a range from one quarter of a millimeter to one millimeter.

The steps of placing, aspirating, and feeding may include improving the efficiency of digestion by removing gas from the stomach and by stepping a feeding material forward from the stomach to a location where greater peristaltic activity facilitates absorption. The method of feeding and decompressing in a gastrointestinal tract may include maximizing feeding while preventing overfeeding by observing a volume of aspirate collected during aspiration, and adjusting the pressure and/or the flow resistance in the feeding channel so that the volume aspirated during a period of time is substantially equal to or less than a volume absorbed during the same period of time.

In still another simple form, a system for aspirating from and feeding into the gastro-intestinal tract in accordance with the present invention may include at least one aspirate reservoir, and at least one aspirate filter in an aspirate line and fluidly connected to the aspirate reservoir. The system may further include at least one one-way valve in the aspirate line, and at least one feeding line fluidly connected to the aspirate reservoir. The one-way valve may be located downstream from the filter. The filter may have a mesh size in a range from one quarter of a millimeter to one millimeter. The system may include a rack that has at least an aspirate reservoir support and a filter support. The filter support may support the filter in a position of use with an outlet of the filter at an upper end of the filter.

The system may further include a combination feeding and aspirate line connected to the aspirate reservoir at one end. The combination feeding and aspirate line may also be connected to each of the aspirating and feeding lines. The aspirating line may include at least one of a nasogastric, nasoenteric, gastrostomy, and jejunostomy tube having aspirating openings adapted for aspiration from the stomach.

The foregoing and other features and advantages of the present invention will be apparent from the following more detailed description of the particular embodiments of the invention, as illustrated in the accompanying drawings wherein
FIG. 1 is a diagrammatic view of a system according to an embodiment of the present invention;
FIG. 2 is an enlarged diagrammatic view of one device useable with the embodiment shown in Figure 1;
FIG. 3 is a schematic representation of the solenoid valve and switch device that may be included in the device of Figures 1 and 2;
FIG. 4 is a diagrammatic view of a device according to a second embodiment of the invention;
FIG. 5 is a diagrammatic view of a device according to a third embodiment of the invention;
FIG. 6 is a sectional view of a filter useable with the device in accordance with the embodiment shown in Figure 5; and
FIG. 7 is an diagrammatic view of a rack in accordance with the present invention.

As discussed above, this invention generally relates to a method and device used to aspirate a gastro-intestinal tract and/or to deliver nutrients, fluids, medication, and/or aspirate into the gastro-intestinal tract. The invention specifically relates to methods and devices for safely, efficiently, and continuously aspirating from the stomach and feeding into the jejunum in the gastro-intestinal tract.

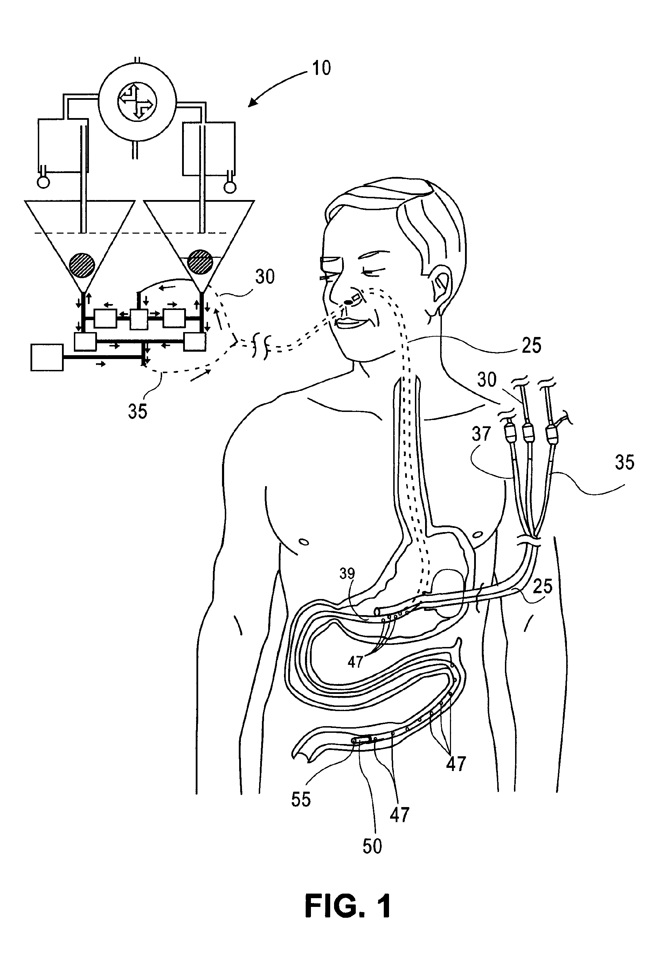

As shown in Figure 1, an aspirate storing and refeeding device 10 is used to feed and decompress in a gastro-intestinal tract 15 of a patient 20. To this end, a combination feeding and aspirating tube 25 may be inserted into the gastro-intestinal tract 15 through the nose and esophagus. Other ways of accessing the gastro-intestinal tract include inserting the tube through the abdominal wall directly into the jejunum, or directly into the stomach by a gastrostomy tube (element 25 shown in solid lines) and intraluminally to the jejunum. As can be appreciated from Figure 1, the combination feeding and aspirating tube 25 may be a double lumen tube and comprises an aspiration channel 30 and a feeding channel 35 for handling flow in opposite respective directions. Combining these channels 30, 35 into a single composite tube 25 has the advantage of reducing bulk and complexity in the inserted portion of the channels 30, 35. However, a separate aspiration channel 37 may be inserted through the abdominal wall as shown or through the nasal passages and esophagus. When a separate aspiration channel 37 is implemented, the feeding line may be a dedicated feeding line.

The aspiration channel 30 may have a distal end 40 including an opening 45 for aspirating fluids including digestive juices and feeding material from the jejunum. Additional openings 47 located at spaced intervals proximally from the distal end 40 along the aspiration channel 30 may be provided. This has the effect of aspirating along a greater length of the gastrointestinal tract as indicated by the collapsed region shown in Figure 1. Additionally or alternatively, openings 47 may be located in the aspiration channel along a length corresponding to a position of the stomach. The distal end 40 of the aspiration channel 30 may be located as indicated at dashed line 49.

The feeding channel 35 may have a distal end 50 extending distally beyond the distal end 40 of the aspiration channel 30. The distal end of the feeding channel 35 may have an opening 55 for delivering feeding material and other fluids as will be described below. The distal end 50 and the opening 55 of the feeding channel 35 may be spaced distally from the distal end 40 and opening 45 of the aspiration channel 30 by approximately ½ inch. Depending on the particulars of the cite at which the channels are placed, the separation of the most distal openings 45, 55 may be in the range from 1/4 inch to 12 inches. In most cases, the separation of the distal openings 45, 55 of the aspirating and feeding channels 30, 35 will not exceed approximately 6 inches.

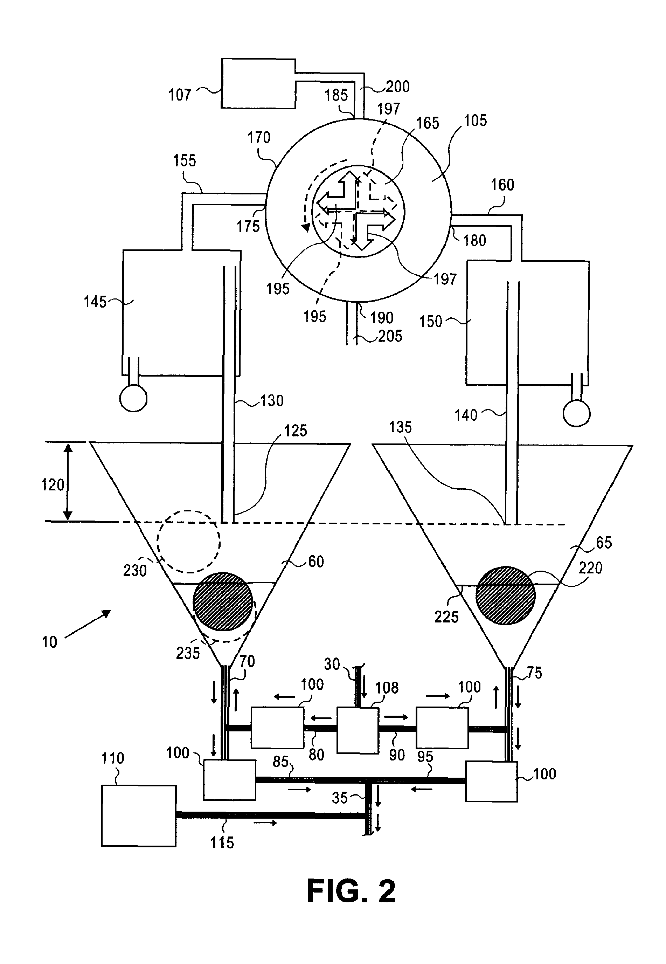

In one embodiment shown in Figure 2, the device 10 comprises a first aspirate reservoir 60 and a second aspirate reservoir 65. The reservoirs 60, 65 can be provided in the form of burettes having single lumen combination feeding and aspirating lines 70, 75 for handling both aspiration and refeeding of aspirate to and from the reservoirs 60, 65, respectively. The combination feeding and aspirating lines 70, 75 generally handle flow in only one direction at a time, either toward or away from the reservoirs 60, 65. However, flow in one of the combination feeding lines is generally in an opposite direction relative to flow in the other of the combination feeding and aspirating lines 70, 75. Thus, there may be an alternating pattern of aspirating into the first reservoir 60 for a first half of a cycle and then aspirating into the second reservoir 65 for a second half of the cycle. Meanwhile, the second reservoir 65 refeeds aspirate for the first half of the cycle and the first reservoir 60 refeeds aspirate for the second half of the cycle. This alternating pattern can be repeated indefinitely during feeding. It is to be understood that refeeding need not be continuous during all of the half cycles. Likewise aspiration flow may cease at any point during each of the half cycles. Additionally, the cycles need not be divided into halves. Rather any alternating pattern of time is considered to be within the scope of the invention.

In order to effectuate the alternating pattern of aspirating and refeeding, the device 10 includes a system of lines 80, 85, 90, 95, one-way valves 100, and a solenoid valve 105 for alternatingly connecting the reservoirs to a vacuum source 107. The one-way valves 100 permit flow in the lines 80, 85, 90, and 95 only in the directions indicated by the arrows in Figure 2. Thus, when the solenoid valve 105 connects either of the reservoirs 60, 65 with the vacuum source 107, only flow through the aspirating lines 80 and 90 is induced, which in turn induces flow from the aspiration channel 30. When the vacuum source 107 is disconnected from one of the reservoirs 60, 65, the contents of that reservoir are permitted to flow out through one of the feeding lines 85, 95, preferably under the influence of gravity. Alternatively, a pump or vacuum source 107 could be connected to the feeding channel 35 to draw and refeed the aspirate into the feeding channel 35. A filter 108 can also be placed in the aspiration channel 30 or between the aspiration channel 30 and the aspirating lines 80, 90 as shown in Figure 2.

As shown, the device 10 may be provided in addition to the feeding system that is typically used in hospitals. As such, a feeding material is provided in a feeding bag 110. The feeding bag may be connected to the feeding channel 35 by an unused feeding material line 115. The flow of the feeding material into the feeding channel 35 may be affected by the capacity of the feeding channel 35, the pressure at which the feeding material is fed from the feeding bag 110, (such as by a pump), and the flow rate of the aspirate that is being refed into the feeding channel 35. These and other factors can be adjusted to adjust the flow of feeding material into the feeding channel. Preferably, the flow from the feeding bag 110 can be reduced in proportion to a flow of aspirate that is refed. Thus, waste of unused feeding material can be avoided. Furthermore, nutrients and digestive juices that typically have been discarded in the past can be reintroduced and absorbed with little or no loss of fluids and nutrients.

The device 10 also enables the aspiration to be applied continuously. That is, vacuum from the vacuum source can be applied substantially constantly. Flow of the aspirate may not be constant or continuous due to a presence or a lack of presence of fluids in the portion of the gastrointestinal tract being aspirated. However, continuous and/or constant aspiration has the advantage of continuously removing gases from the gastrointestinal tract as there is no interval without suction during which gas may be propelled by peristalsis to a location distal to the most distal aspiration orifice. Removing these gases has the advantages set forth above. Furthermore, removing the gases continuously or constantly has the advantage of preventing distending of the intestines due to build up of these gases.

When the length of the half cycles are set properly, both refeeding flow and aspiration flow can be continuous, if not constant. With more constant and/or continuous flow in the feeding channel, the fluctuation of flow into the gastrointestinal tract may be reduced. By providing less fluctuation in the flow into the intestines, distending can further be reduced or prevented. Furthermore, nutrients and fluids will be more constantly available for absorption by the intestines.

A predetermined volume of aspirate to be retained in the reservoirs 60, 65 can be selected and set. This volume also affects the "head" or pressure due to gravitational forces that causes the flow of aspirate out of the reservoirs 60, 65. A maximum predetermined volume to be retained in each reservoir 60, 65 during each half cycle may be in the range from 10 to 15 cc. This reduces the chance for overfeeding, for example, when the half cycle is kept to approximately ½ minute. Perhaps a maximum adjustable volume could be as much as 35 cc. Adjustment of the predetermined volume may be achieved by adjusting the depth 120 of a lower end 125 of a first draw tube 130 in the first aspirate reservoir 60. Similarly, the maximum volume of aspirate to be retained in the second aspirate reservoir 65 may be adjusted by adjusting a depth 120 of a lower end 135 of a similar second draw tube 140.

The draw tubes 130, 140 connect the aspirate reservoirs 60, 65 to first and second overflow chambers 145, 150. These tubes also provide fluid communication between an interior of each of the reservoirs 60, 65 and the solenoid valve 105. Thus, draw tubes 130, 140 connect the aspirate reservoirs to the vacuum source 107 in an alternating pattern as will be described in further detail below. The draw tubes 130, 140 act to remove aspirate in excess of the predetermined maximum volume and draw the excess aspirate into respective overflow chambers 145, 150. The overflow chambers 145, 150 may have any reasonable volume. The volume of each overflow chamber may be in the range from 200-4000 cc, with 1000 cc being a fairly standard capacity. Once removed, the excess aspirate can be discarded or refed later when the flow of aspirate from the aspiration channel 30 has decreased.

As shown in Figure 2, the first and second overflow chambers 145, 150 are connected to the solenoid valve by first and second valve connection lines 155, 160. Thus, the overflow chambers 145, 150 are fluidly connected to the solenoid valve 105 and to the reservoirs 60, 65 via the draw tubes 130, 140. The solenoid valve may comprise a single motive member 165 contained within a solenoid housing 170. The motive member 165 provides at least two (2), two-way valves for connection between at least four ports 175, 180, 185, and 190. Preferably, the motive member has first and second channels 195 and 197. The first channel 195 connects the first valve connection line 155 from the first aspirate reservoir 60 to the vacuum source 107 through a vacuum line 200 or to an ambient pressure volume through a vent hose 205. The second channel of the motive member 165 connects the second valve connection line 160 to the ambient pressure volume through the vent line 205 or to the vacuum source 107 through the vacuum line 200. In one configuration of the solenoid valve, the motive member 165 is a rotary member that rotates under the influence of a solenoid through a range of approximately ninety degrees to exclusively connect either the first aspirate reservoir 60 or the second aspirate reservoir 65 to the vacuum source 107 as shown in Figure 2. Alternatively stated, the first channel either connects the first reservoir 60 to the vacuum line 200 or to the vent line 205. Likewise, the second channel 197 of the motive member 165 either connects the second aspirate reservoir 65 to the vent line 205 or to the vacuum line. Only one of the reservoirs 60, 65 is connected to the vacuum source 107 at a time. The other reservoir is connected to ambient air through the vent line 205. By way of example and not by way of limitation, this may be efficiently achieved by a single motive member that rotates or moves linearly between only two operating positions.

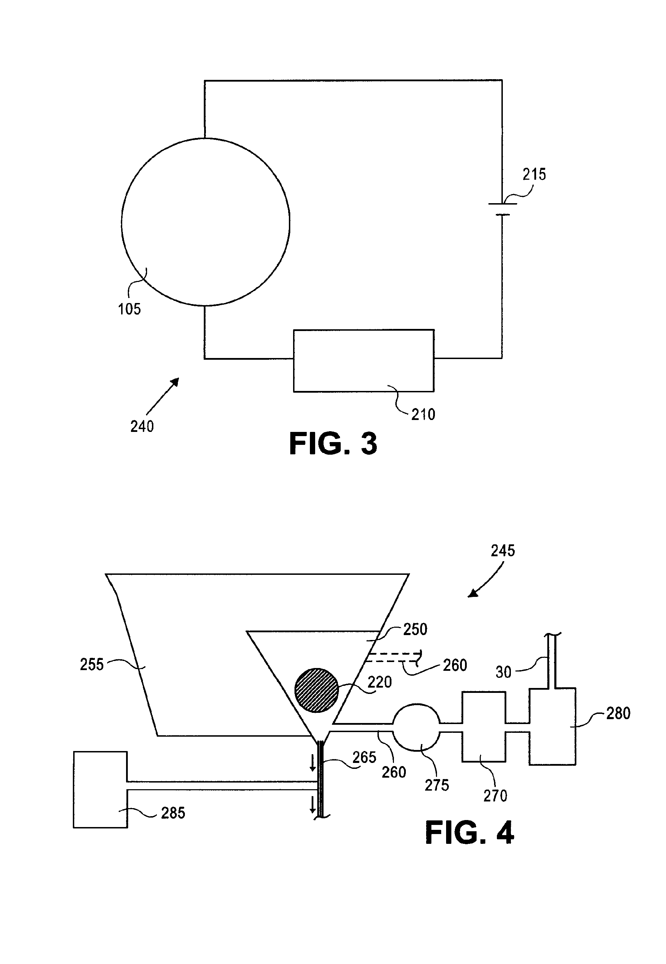

The connections to the vacuum source 107 and vent 205 are alternated back and forth so that one of the aspirate reservoirs 60, 65 is connected to the vacuum source 107 and the other is connected to the vent at all times. This may be achieved by a switch connected to the solenoid valve 105, and wherein the solenoid valve is in a first position when the switch is closed and in a second position when the switch is opened. That is, applying power to the solenoid valve places it in a first position and removing power from the solenoid valve places it in a second position indicated by the dashed arrows in Figure 2. The switch may be a timer switch 210 in a circuit connected to a power source 215 as shown in Figure 3. The timer switch 210 can be adjusted to actuate its switch at a predetermined interval corresponding to a half cycle. Thus, the switch 210 will be turned on or off after each half cycle and the motive member 165 will be moved to its first or second position.

The solenoid could be replaced by a different solenoid valve. For example, the solenoid valve may comprise a piston member and more than four ports. The solenoid valve can connect the aspirate reservoirs to respective sources of vacuum and/or to respective vent lines. Further alternatively, the device could be manually operated. That is, instead of employing a timer switch, a switch could be operated by hand. Further alternatively, manually operated valves could be provided so that a person would be required to switch the connections to the reservoirs back and forth between vacuum and vent.

As indicated above, the amount of aspirate received into the reservoirs 60, 65 optimally is equal to or slightly less than the amount refed during a half cycle of the device. However, the amount refed varies during feeding and over the course of recuperation. Therefore, there will be periods of time during some half cycles when the aspiration is being applied, but little or no aspirate is flowing. Likewise there will be periods when the aspirate being refed is completely withdrawn from a reservoir before a current half cycle has ended. Since a reservoir is connected to ambient air pressure during feeding from that reservoir, there is a potential for drawing air into the feeding channel and eventually into the gastrointestinal tract. To prevent this from happening, a check valve is provided in each reservoir 60, 65 by a ball 220. As shown, the ball 220 is buoyant and is thus positioned by the aspirate at a first level 225. When the aspirate reaches a maximum level, the ball floats at the maximum aspirate level as indicated at 230. However, when the aspirate completely leaves the reservoirs 60, 65, the ball 220 seats in a sealed position as shown at 235. With aspirate refeed being induced by gravity, this type of check valve will normally be sufficient to prevent air from being drawn into the feeding channel 35. On the other hand, if a positive pressure pump is employed to draw and refeed the aspirate, a more positive shutoff valve may be necessary.

As can be appreciated, the device 10 may include a solenoid valve and timer device 240 as shown in Figure 3. Furthermore, the solenoid valve and timer device 240 have characteristics that may be beneficial when used with the overall device 10 described above. Specifically, the solenoid valve 105 could be made automatic by an electrical circuit including a timer switch 210. The timer switch can comprise microcircuitry that includes a timer. The timer switch 210 can further include low voltage switch actuation that opens and closes the circuit connected to the power source 215. Alternatively, the timer switch can be made up of discrete electrical components that accomplish the same alternating timing function. Further alternatively, the timer switch can include a combination of electrical and mechanical elements that achieve the same alternating timing function.

Figure 4 is a diagrammatic view of a device 245 according to a second embodiment of the invention. This device 245 differs from the device 10 described above in that the previously described device had two aspirate reservoirs whereas the device 245 has only one aspirate reservoir 250. The aspirate reservoir 250 in this case has a capacity equal to a maximum volume of aspirate to be retained in the reservoir. Any excess aspirate will spill into an overflow chamber 255. A further difference is that the line connected to the reservoir 250 for refeeding aspirate does not also function to conduct aspirate to the reservoir 250. That is, the aspirate channel 30 is connected to the reservoir by an aspirating line 260 and the feeding channel 35 is connected to the reservoir 250 by an aspirate refeeding line 265. As such, aspiration and refeeding can occur simultaneously from the same reservoir 245. However, aspiration cannot be effectuated by a vacuum source in fluid communication with the aspiration channel via the reservoir 245. If so, refeeding in the refeeding line 265 would be impeded. Therefore, a positive pressure pump 270 is provided in the aspirating line to draw aspirate toward the reservoir 245. Aspirate can be refed by a gravitational force. However, a pressure source such as a pump could be substituted. To assure flow in the right direction, a one way valve 275 is also placed in the aspirating line 260. As in the previous embodiment a filter 280 is also preferably placed in the aspirating line 260, or between the aspirating line 260 and the aspiration channel 30. A check valve including a ball 220 like that described in the embodiment above is provided in the reservoir 250 of the device 245. This check valve functions substantially the same as that described above. Typically, the reservoir is open to the ambient air so that aspirate can be refed by gravity. Like the previous embodiment, the device is intended to be used in addition to a supply of an unused feeding material that can be fed from a feeding bag 285 by a pump or by gravity.

The device is similar to the previously described embodiment in that it provides for continuous or constant aspiration and refeeding of aspirate. While the device 245 requires a positive pressure source 270, device 245 does not require the solenoid and timer device 240 of the previously described embodiment of Figures 1-3.

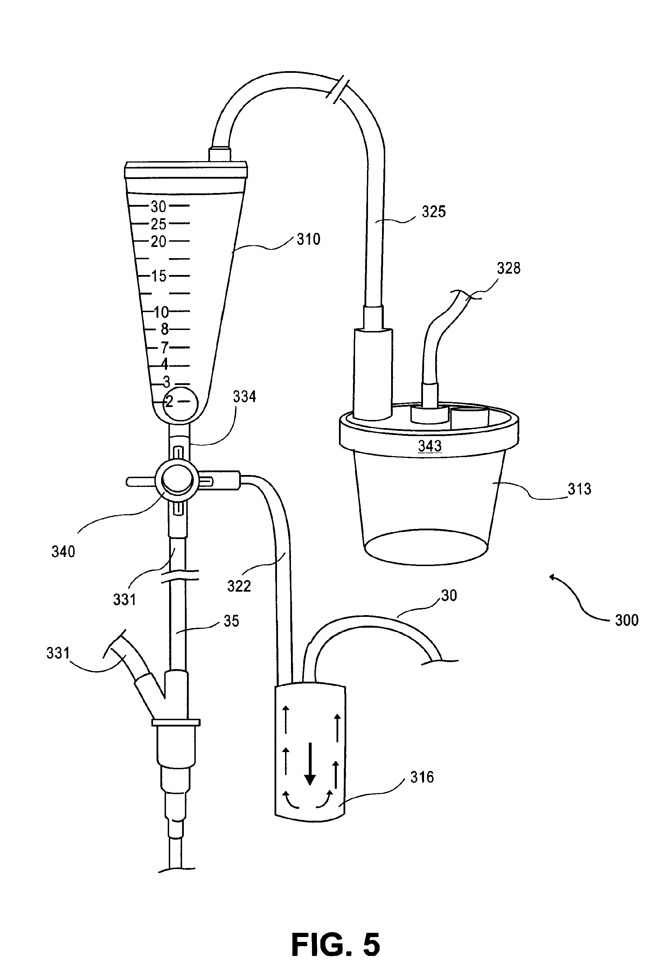

Figure 5 is a diagrammatic view of a device 300 in accordance with a third embodiment of the invention. The device 300 may be used in place of all or part of the devices shown in Figures 1, 2 and 4. For example, the device 300 has an aspirate reservoir 310, an overflow chamber 313, a filter 316 and a one way valve 319 each of which may replace the elements called by the same name in the description of Figures 1, 2, and 4. That is, the aspirate line 30 may be connected to the filter 316. One or more aspirating lines 322 may be connected between the filter 316 and a respective one-way valve 319. Figures 1 and 2 show the filter connected to two one way valves. Similarly, the filter 316 of Figure 5 could be connected to two one-way valves in respective aspirating lines connecting the filter to respective aspirate reservoirs similar to aspirate reservoir 310. Alternatively, the system could have respective filters and one way valves in a pair of aspirating lines fed by aspiration channel 30. Further alternatively, the device 245 shown and described with regard to Figure 4 may be replaced by the device 300 of Figure 5. The similarities between the device 300 and the devices of Figures 1,2 and 4 may include a draw tube 325 similar to draw tubes 130, 140, a vacuum/valve connection line 328 similar to valve connection lines 155, 160, and an unused material feeding line 331 similar to the feeding line 115. A combination feeding and aspirating line 334 may be similar to either one of combination feeding and aspirating lines 70, 75. A feeding line 337 may be similar to the feeding lines 85, 95, or could be analogous to the feeding channel 35 if the feeding line 337 is used by itself.

As may be appreciated, the device 300 may be used with or without a solenoid valve similar to that shown and described with regard to Figures 1-2. Alternatively, a vacuum source may be connected to the vacuum/valve connection line 328 for controlled application of vacuum for cycling for non-continuous flow from a device incorporating only one aspirate reservoir 310 in accordance with the elements shown in figure 5. The device 300 or a device 10 adapted to include the elements of device 300 may further include a stop cock 340 for manually stopping flow in any one of the three lines 322, 334, and 337 that form a junction at the stop cock 340.

Another difference shown in Figure 5 is that the overflow chamber 313 is connected via a flexible draw tube 325 and has the draw tube connected via an apertured lid 343. Thus, the tubes/lines 325, 328 may be easily connected and/or disconnected. The filter 316 incorporated with the device 300 of Figure 5 may also be configured for use in specific applications. For example, in applications in which greater amounts of gas will be aspirated, the orientation of the filter may become important. The filter 316 shown in Figure 5 is configured for use in aspirating from a stomach where the amount of air/gas to be aspirated is likely to be greater than in other parts of the gastro-intestinal tracts. For this application, both the input and outlet ports are located in an upper portion of the filter 316.

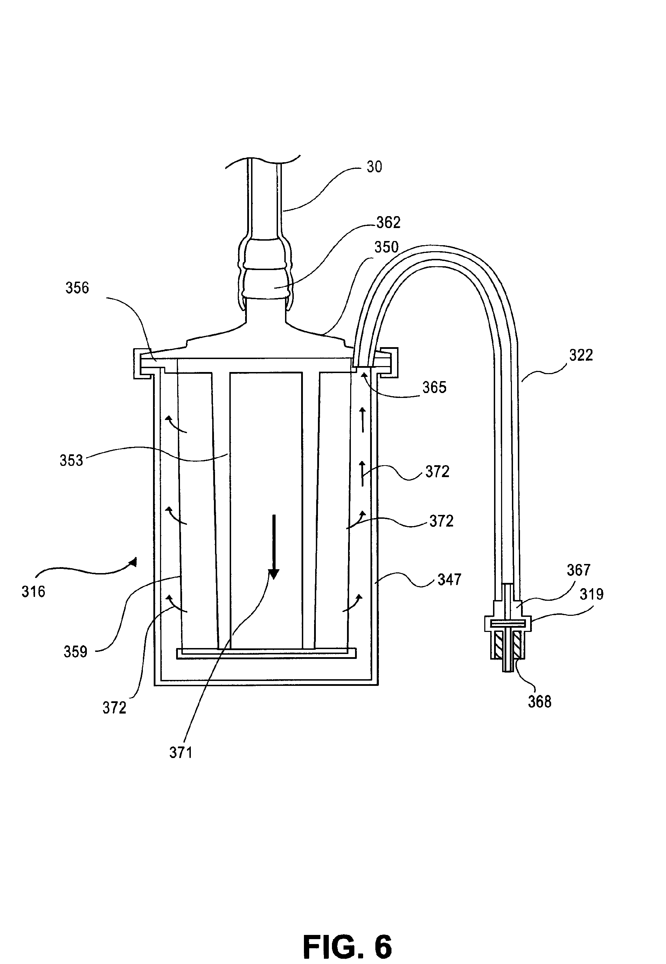

Figure 6 is a sectional view taken through a central vertical plane of the filter 316 of Figure 5. The filter may include a canister 347 and a lid 350 for encapsulating a filter core including a frame 353 with a flange 356 that may be captured between the lid 350 and the cannister 347. A filter membrane 359 may be supported on the frame 353 in a manner that no fluid may pass through the cannister 347 except by passing through the membrane 359. While such filters are known for other applications, this filter 316 is configured for inhibiting solids greater than a predetermined size from passing through the filter 316. Thus, the membrane 359 may be provided with a mesh having membrane openings with dimensions in a range from one quarter millimeter to one millimeter to only permit particles of the opening size or smaller to pass through the filter 316. This range of sizes is advantageously adapted to allow for feeding of relatively course material while inhibiting mucous or other unwanted matter to pass through the filter.

In use, an aspirate may be drawn from the stomach and/or jejunum through the aspiration channel 30, into a port formed by a nipple 362 of the lid 350 at or near the top of the filter 316. The fluid aspirate will be drawn downward into the cannister 347. Any air will remain or bubble to an upper portion of an interior volume formed by the filter cannister 347 and lid 350. If the cannister is maintained in an upright orientation, the air will be drawn out of the filter 316 through an exit port 365 formed in the lid 350 near an upper portion of the cannister 347 and lid 350 prior to any liquid aspirate material being drawn out. In this way an undesirable build up of air may be avoided. As a result, the flow of fluid aspirate can be kept more consistent and excess bubbling, and associated vibrations can be avoided.

As shown in Figure 6, the aspirating line 322 may be connected to the outlet port 365. The one-way valve 319 may be placed in the aspirating line 322 to prevent back flow of the aspirate. As shown, the one-way valve 319 may be provided together with a connection fitting 367. The aspirating line 322 may be connected to a downstream end of the connection fitting 367, and an upstream end of the fitting 367 may have luer threads 368 for connection to the combination feeding and aspirating line 334 via the stop cock valve 340, as shown in Figure 5 for example. The one-way valve permits flow in a direction of the arrows 371, 372 in Figure 6, and inhibits flow of the fluid aspirate in a direction opposite to that of the arrows.

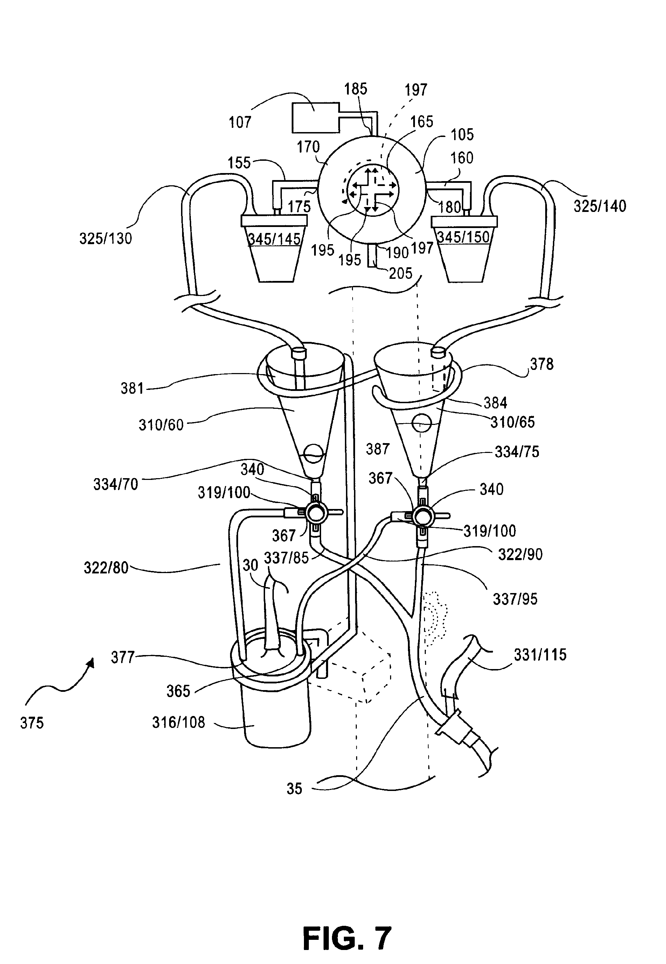

Figure 7 shows a system 375 similar to that of Figure 1 and further incorporating one or more of each of the elements of the device 300 of Figure 5. As shown, by implementing two aspirate reservoirs 310 and two overflow chambers 345, a configuration like that of Figure 1 may be achieved. To achieve a single filter similar to that of Figure 1, two aspirating lines 337 may be connected at respective exit ports 365 and 376. Otherwise, the system of Figure 7 may be a combination of some of the details of Figure 6 with a system of Figure 1. A valve for switching vacuum pressure from one overflow chamber 345 and one aspirate reservoir 310 to the other overflow chamber 345 and aspirate reservoir 310 may be implemented similar to the valve implemented with the embodiment of Figure 1. This valve may be a solenoid valve 105 that may operate substantially similarly to that described above.

The system 375 may further include a rack 378 having two upper loops 381 and 384 formed by an upper S-configured portion for supporting respective ones of the aspirate reservoirs 310. The upper S-configured portion may be supported by a vertical extension 387. A lower loop 390 may extend from a lower end of the vertical extension 387 and provide support for the filter 316. A mounting stud 393 may extend downwardly from the lower loop 390 for engagement in a platform 396 of an IV pole, for example. The valve 105, overflow chambers 325, and any other associated components may also be supported on the IV pole as desired. The numbering of elements in Figure 7 is shown in the alternative by slashes indicating that the system 375 of Figure 7 may be just one exemplary embodiment of one or more systems in accordance with the systems illustrated in Figure 1. It is to be understood that any of the systems and methods described with regard to any of the embodiments of the present invention may be combined in any manner with any other systems and methods of the invention without departing from the spirit and scope of the invention.

It is to be understood, that the filter 316 could be placed on its side or otherwise oriented differently than the vertical position without departing from the spirit and scope of the invention. However, having at least an outlet port at an upper end of the filter encourages any gas to exit the filter first. Furthermore, having both the input and exit ports at or near the top of the filter shortens the path that gases or air have to travel when they must pass through the filter. If such gases must pass upwardly through liquid aspirate, there will be some competition between the liquid and the gases moving through the exit port. It is also more likely that larger, less consistent amounts of liquid aspirate will be forced by larger bubbles through the aspirating line 322. This may result in a less controlled and less constant delivery of aspirate to the aspirate reservoir and possibly less constant delivery of refeeding material to a patient.

Overall, it is to be understood that the present invention, among other things, if for aspirating from a stomach and refeeding aspirate taken from the stomach back into the jejunum of the gastro-intestinal tract. This has the advantage of stepping the feeding material further forward in the digestive tract and the advantage of removing a larger amount of air that is more typically to be found in the stomach.

It is to be understood that the term positive pressure source generally refers to a pump or an equivalent, such as a syringe or pressurized receptacle. The term "positive" refers to an "active" as opposed to a passive pressure means, such as a gravitational pressure means. It is also to be understood that vacuum source refers to any number of equivalents including but not limited to a pump, an aspirating syringe, or a negative pressure receptacle. One common vacuum source is that typically provided by a hospital or other facility and piped throughout the facility to the various stations including patients' rooms.

The embodiments and examples set forth herein were presented in order to best explain the present invention and its practical application and to thereby enable those of ordinary skill in the art to make and use the invention. However, those of ordinary skill in the art will recognize that the foregoing description and examples have been presented for the purposes of illustration and example only. The description as set forth is not intended to be exhaustive or to limit the invention to the precise form disclosed. Many modifications and variations are possible in light of the teachings above without departing from the spirit and scope of the forthcoming claims.

## Claims

1. Apparatus for feeding into and decompressing a gastrointestinal tract, comprising:
a feeding channel for feeding into a jejunum;
an aspiration channel for aspirating a stomach; and
a refeeding device coupled to the feeding channel and the aspiration channel for refeeding aspirate that has been removed from the stomach through the aspiration channel, through the feeding channel.

2. A system for aspirating from and feeding into the gastro-intestinal tract, comprising:
at least one aspirate reservoir;
at least one aspirate filter in an aspirate line and fluidly connected to the aspirate reservoir;
at least one one-way valve in the aspirate line; and
at least one feeding line fluidly connected to the aspirate reservoir.

3. The system of claim 2, wherein the one-way valve is located downstream from the filter.

4. The system of claim 2 or 3, wherein the filter has a mesh size in a range from one quarter of a millimeter to one millimeter.

5. The system of claim 2, 3 or 4, wherein the filter has an outlet, the system further comprising a rack having at least an aspirate reservoir support and a filter support, wherein the filter support locates the outlet of the filter at an upper end of the filter in a position of use.

6. The system of any one of claims 2 to 5, further comprising a combination feeding and aspirating line connected to the aspirate reservoir at on end, the combination feeding and aspirating line also being connected to each of the aspirating and feeding lines.

7. The system of any one of claims 2 to 6, wherein the aspirate line comprises at least one of a nasogastric, nasoenteric, gastrostomy, and jejunostomy tube having aspirating openings adapted for aspiration from the stomach.

8. A method of feeding and decompressing in a gastrointestinal tract, comprising the steps of:
feeding through a feeding channel into a jejunum;
aspirating through an aspiration channel from a stomach;
wherein the step of feeding comprises refeeding aspirate that has been removed from the stomach in the step of aspirating.

9. The method of feeding and decompressing of claim 8, wherein the step of aspirating comprises aspirating from the jejunum and the stomach.

10. The method of feeding and decompressing of claim 8 or 9, further comprising:
placing a distal end of a feeding channel at a first position in a jejunum;
placing a distal end of an aspiration channel at a second position in the stomach; and
aspirating via the aspiration channel;

11. The method of feeding and decompressing of any one of claims 8 to 10, further comprising the steps of:
placing a distal end of an aspiration channel at a second position in the jejunum spaced proximally relative to the first position; and
continuously aspirating and substantially collapsing a portion of a jejunum at the second position.

12. The method of claim 8, wherein the step of feeding and the step of aspirating comprise feeding and aspirating from separate tubes forming separate channels.
Docket No. MOSS-10654

13. The method of claim 8, wherein the step of feeding and the step of aspirating comprise feeding and aspirating through a single composite line having plural channels.

14. A method of feeding and decompressing in a gastrointestinal tract, comprising the steps of:
placing a distal end of a feeding channel at a first position in the jejunum;
placing a distal end of an aspiration channel at a second position in the stomach; and
continuously aspirating via the aspiration channel;
wherein the steps of continuously aspirating and continuously feeding further comprise alternatingly aspirating into a plurality of aspirate reservoirs in a repeated cycle.

15. The method of feeding and decompressing in a gastrointestinal tract of claim 14, further comprising continuously feeding via the feeding channel.

16. The method of feeding and decompressing of claim 14, further comprising feeding from one of the plurality of aspirate reservoirs into which nothing is being aspirated.

17. The method of feeding and decompressing of claim 14, wherein the step of continuously aspirating further comprises:
aspirating an amount of aspirate greater than a predetermined maximum into a first overflow chamber connected to the first reservoir during a first part of a cycle; and
aspirating an amount of aspirate greater than the predetermined maximum into a second overflow chamber connected to the second reservoir during a second part of the cycle.

18. A method of feeding and decompressing in a gastrointestinal tract, comprising the steps of:
placing a distal end of a feeding channel at a first position in the jejunum;
placing a distal end of an aspiration channel at a second position in the stomach;
aspirating via the aspiration channel;
Docket No. MOSS-10654
feeding via the feeding channel; and
inhibiting gastric reflux without permanently removing natural gastric juices.

19. The method of claim 18, further comprising reducing one or more of dehydration, loss of natural antibodies, and lose of enzymes, wherein the step of feeding comprises refeeding a feeding material removed during the step of aspirating.

20. The method of claim 18, further comprising filtering an aspirate from the stomach to a maximum particle size in a range from one quarter of a millimeter to one millimeter.

21. The method of feeding and decompressing in a gastrointestinal tract of claim 18,
wherein the steps of placing, aspirating, and feeding further comprise improving the efficiency of digestion by removing gas from the stomach and by stepping a feeding material forward from the stomach to a location where greater peristaltic activity facilitates absorption.

22. The method of feeding and decompressing in a gastrointestinal tract of claim 18, further comprising maximizing feeding while preventing overfeeding by:
observing a volume of aspirate collected during aspiration; and
adjusting the pressure or the flow resistance in the feeding channel so that the volume aspirated during a period of time is substantially equal to or less than a volume absorbed during the same period of time.
